# EUROPEAN PATENT APPLICATION

(11) **EP 2 368 870 A1**
(43) Date of publication of application: **28.09.2011**
(21) Application number: 09831928.8
(22) Date of filing: 09.12.2009
(51) Int. Cl.: C07C 51/41, C07B 57/00, C07C 51/02, C07C 51/09, C07C 51/43, C07C 51/493, C07C 61/22

(54) **PROCESS FOR PRODUCING OPTICALLY ACTIVE CARBOXYLIC ACID**

(30) Priority: 12.12.2008 JP 2008316849
(71) Applicant: Daiichi Sankyo Company, Limited, Chuo-ku Tokyo 103-8426 (JP)
(72) Inventor: SATO, Koji, Tokyo 134-8630 (JP); KUBOTA, Kazuo, Tokyo 134-8630 (JP)
(74) Representative: Smyth, Gyles Darren
(86) International application number: PCT/JP2009/070613
(87) International publication number: WO 2010/067824

(57) **Abstract**

It has been demanded to provide a process for industrially producing an intermediate for a compound that exhibits an inhibitory effect on activated blood coagulation factor X and is useful as a preventive and/or therapeutic agent for thrombotic diseases. The present invention provides a process for producing the (R-α-phenylethylamine salt of (S)-3-cyclohexene-1-carboxylic acid, comprising reacting 3-cyclohexene-1-carboxylic acid and (R)-α-phenylethylamine using a mixed solvent of water and acetone or a mixed solvent of water and ethyl acetate as a solvent.

## Description

### Technical Field

The present invention relates to a process for producing an intermediate for a compound that exhibits an inhibitory effect on activated blood coagulation factor X (also referred to as activated factor X or FXa), and is useful as a preventive and/or therapeutic drug for thrombotic diseases.

### Background Art

For example, N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide represented by the following formula (X): 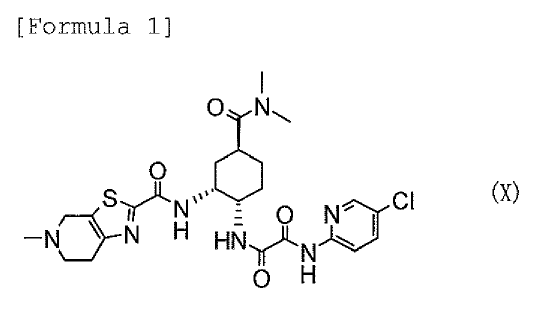 or a salt thereof, or a hydrate thereof, for example, the p-toluenesulfonic acid monohydrate of compound X represented by the following formula (Y): 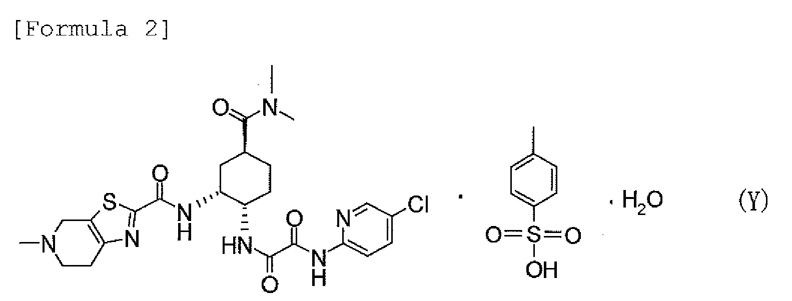 is known as a compound that exhibits an inhibitory effect on activated blood coagulation factor X (also referred to as activated factor X or FXa), and is useful as a preventive and/or therapeutic drug for thrombotic diseases (Patent Literature 1 to 4).

(S)-3-Cyclohexene-1-carboxylic acid represented by the following general formula (A) (hereinafter, also referred to as compound A): 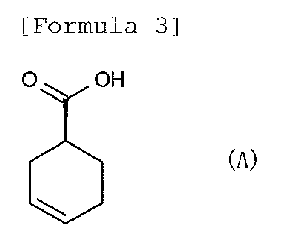 is known as an intermediate for these FXa inhibitory compounds (Patent Literature 1 to 4).

It is known that compound A is obtained by optical resolution of 3-cyclohexene-1-carboxylic acid (hereinafter, also referred to as compound I) using (R)-α-phenylethylamine (hereinafter, also referred to as (R)-PEA) (Non Patent Literature 1). Non Patent Literature 1 makes no mention about solvents used in the optical resolution and discloses that as many as 5 or more recrystallization steps are required.

It has also been reported that compound A is obtained by an asymmetric hydrolysis reaction with an enzyme (Non Patent Literature 2). However, this method requires large amounts of solvents. Thus, an efficient method has been essential from the viewpoint of volumetric efficiency with industrial production in mind. Furthermore, the methods of Non Patent Literature 1 and 2 generate a stereoisomer (R)-3-cyclohexene-1-carboxylic acid as a by-product. However, neither of these documents discloses a method for recycling this.

Moreover, a process has also been reported which comprises stereoselectively obtaining compound A by an asymmetric Diels-Alder reaction using D-pantolactone as an asymmetric auxiliary group (Non Patent Literature 3). However, D-pantolactone is expensive. Thus, a more inexpensive process has been demanded with industrial production in mind.

### Citation List

### Patent Literature

Patent Literature 1: International Publication No. WO 03/000657
Patent Literature 2: International Publication No. WO 03/000680
Patent Literature 3: International Publication No. WO 03/016302
Patent Literature 4: International Publication No. WO 04/058715

### Non Patent Literature

Non Patent Literature 1: Harold M. Schwartz et al., J. Am. Chem. Soc., 100, 5199-5203, 1978
Non Patent Literature 2: Cihangir Tanyeli et al., Tetrahedron: Asymmetry, 15, 2057-2060, 2004
Non Patent Literature 3: Barry M. Trost et al., Tetrahedron Lett., 32, 1613-1616, 1991

### Summary of Invention

### Technical Problem

An object of the present invention is to provide a process for inexpensively and efficiently producing the (R)-α-phenylethylamine salt of (S)-3-cyclohexene-1-carboxylic acid or (S)-3-cyclohexene-1-carboxylic acid from 3-cyclohexene-1-carboxylic acid.

### Solution to Problem

As a result of conducting diligent studies to attain the object, the present inventors have found that: surprisingly, the use of aqueous acetone or aqueous ethyl acetate as a reaction solvent and a recrystallization solvent in the step of obtaining the (R)-α-phenylethylamine salt of (S)-3-cyclohexene-1-carboxylic acid from 3-cyclohexene-1-caxboxylic acid can efficiently produce a highly pure (R)-α-phenylethylamine salt of (S)-3-cyclohexene-1-carboxylic acid; and the stereoisomer (R)-3-cyclohexene-1-carboxylic acid obtained in this step is racemized to racemic 3-cyclohexene-1-carboxylic acid, which can in turn be recycled in the production of the (R)-α-phenylethylamine salt of (S)-3-cyclohexene-1-carboxylic acid or (S)-3-cyclohexene-1-carhoxylic acid. Based on these findings, the present invention has been completed.

### Advantageous Effects of Invention

The present invention provides a process for inexpensively and efficiently producing the (R)-α-phenylethylamine salt of (S)-3-cyclohexene-1-carboxylic acid and/or (S)-3-cyclohexene-1-carboxylic acid. The present invention further provides a process for racemizing, to 3-cyclohexene-1-carboxylic acid, an unnecessary stereoisomer (R)-3-cyclohexene-1-carboxylic acid formed in obtaining (S)-3-cyclohexene-1-carboxylic acid from 3-cyclohexene-1-carboxylic acid.

### Description of Embodiments

Specifically, the present invention relates to:
[1] a process for producing a compound represented by the general formula (II): 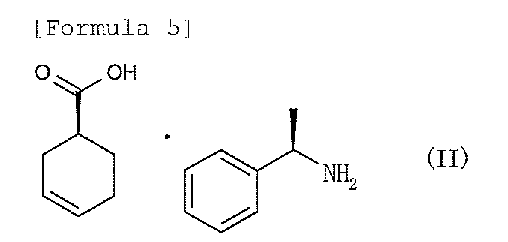 the process comprising reacting a compound represented by the general formula (I): 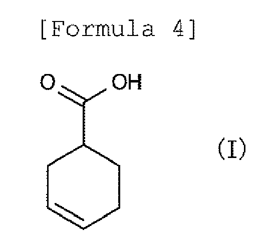 with (R)-α-phenylethylamine using aqueous acetone or aqueous ethyl acetate as a solvent;
[2] the process according to [1], further comprising the step of recrystallizing the compound represented by the general formula (II) using aqueous acetone or aqueous ethyl acetate as a recrystallization solvent;
[3] the process according to [2], wherein aqueous ethyl acetate is used as the solvent and the recrystallization solvent;
[4] the process according to [3], wherein the aqueous ethyl acetate has a water content of 0.5% to 3.0%;
[5] the process according to any one of [1] to [4], further comprising the step of treating the compound represented by the general formula (II) with an acid to obtain a compound represented by the general formula (A): 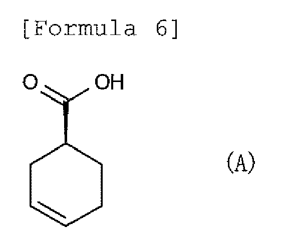
[6] a process for producing a compound represented by the general formula (I): 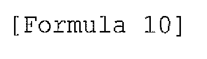 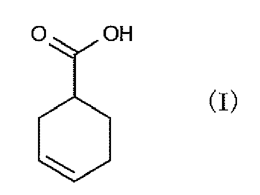 the process comprising: reacting a stereoisomer represented by the general formula (III): 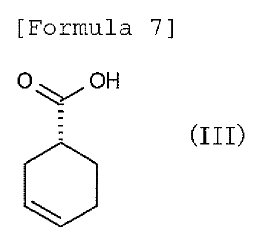 obtained in a production process according to [1], with a C1 to C6 alkyl alcohol in the presence of an acid catalyst;
   reacting the obtained compound represented by the general formula (IV): 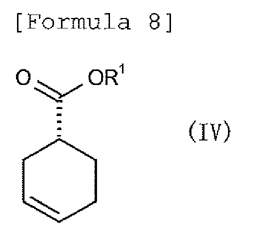 (wherein R¹ represents a C1 to C6 alkyl group) with a base in a solvent to obtain an ester represented by the general formula (V): 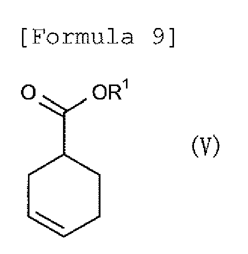 (wherein R¹ is as defined above); and
   hydrolyzing the ester in a C1 to C6 alkyl alcohol;
[7] the process according to [6], wherein the solvent used in the step of obtaining the compound represented by the general formula (V) from the compound represented by the general formula (IV) is N,N-dimethylformamide;
[8] the process according to [6] or [7], wherein the base used in the step of obtaining the compound represented by the general formula (V) from the compound represented by the general formula (IV) is 1,8-diazabicyclo[5.4.0]undec-7-ene;
[9] the process according to any one of [6] to [8], wherein the solvent used in the hydrolysis is methanol or ethanol;
[10] the process according to any one of [6] to [9], wherein a base used in the hydrolysis is sodium hydroxide;
[11] a process for producing a compound represented by the general formula (I): 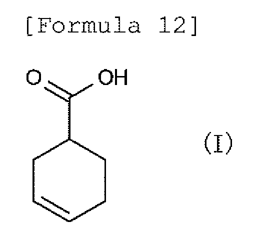 the process comprising reacting a stereoisomer represented by the general formula (II): 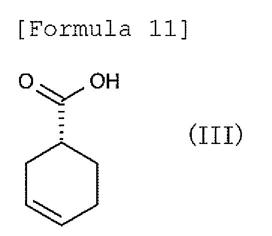 obtained in a production process according to [1], with a base in a solvent;
[12] the process according to [11], wherein the solvent is N,N-dimethylformamide; and
[13] the process according to [11] or [12], wherein the base is sodium hydride.

In the present specification, "C1 to C6 alkyl" refers to a linear or branched alkyl group having 1 to 6 carbon atoms. Examples of the C1 to C6 alkyl include methyl, ethyl, propyl, and isopropyl.

In the present specification, examples of the "C1 to C6 alkyl alcohol" include methanol, ethanol, propanol, and isopropyl alcohol.

In the present specification, "aqueous solvent" refers to a mixed solvent of water and a solvent other than water. Mixing of water and the solvent other than water may be performed before or during the reaction, and is not particularly limited as long as it is performed in an environment where water and the solvent other than water act as solvents.

In the present specification, "equivalent" means a molar equivalent unless otherwise specified.

N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide represented by the following formula (X): 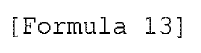 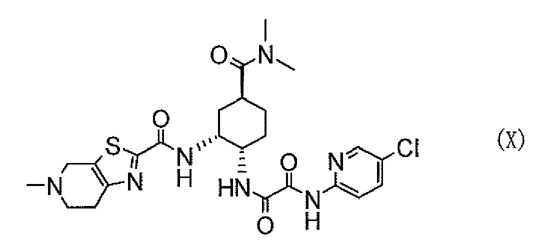 is the free form of the compound represented by the formula (Y), and has been registered as International Nonproprietary Name (INN): edoxaban, (N-(5-chloropyridin-2-yl)-N'-[(1S,2R,4S)-4-(N,N-dimethylcarbamoyl)-2-(5-methyl-4,5,6,7-tetrahydro[1,3]thiazolo[5,4-c]pyridine-2-carboxamido)cyclohexyl]oxamide) in the World Health Organization (WHO).

Hereinafter, the process of the present invention will be described in detail. 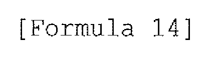 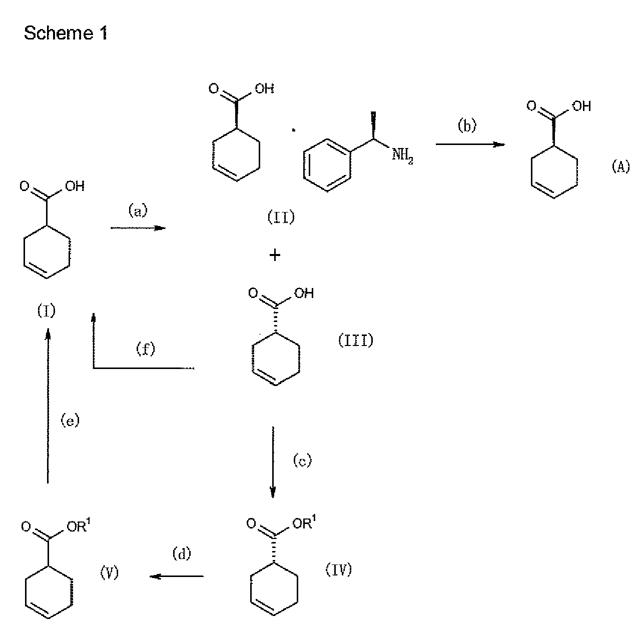 (wherein R¹ represents a C1 to C6 alkyl group)

### (Step a)

The compound represented by the general formula (II) (hereinafter, also referred to as compound II) can be obtained as a crystalline diastereomeric salt by allowing (R)-PEA as an optically active base to act on compound I in a solvent. Recrystallization of this salt can be further repeated to obtain a more highly pure compound II (Step a).

Compound I and (R)-PEA can be synthesized by processes known in the art, or may be purchased from a distributor.

Examples of the solvent used in the salt resolution include, but are not particularly limited to: water; alcohol solvents such as methanol, ethanol, and isopropyl alcohol; ether solvents such as diethyl ether, dipropyl ether, diisopropyl ether, tetrahydrofuran, methyl t-butyl ether, and cyclopentyl methyl ether; ester solvents such as methyl formate, ethyl formate, methyl acetate, ethyl acetate, propyl acetate, butyl acetate, and phenyl acetate; halogenated hydrocarbon solvents such as dichloromethane, chloroform, carbon tetrachloride, dichloroethane, and tetrachloroethane; ketone solvents such as acetone, methyl ethyl ketone, diethyl ketone, and methyl isobutyl ketone; aromatic hydrocarbon solvents such as benzene, chlorobenzene, toluene, and xylene; and nitrogen-containing solvents such as acetonitrile, N,N-dimethylformamide, N,N-dimethylacetamide, and N-methylpyrrolidone. The solvent is preferably water, methanol, ethanol, isopropyl alcohol, diisopropyl ether, ethyl acetate, chloroform, acetone, toluene, acetonitrile, or a mixed solvent thereof, more preferably ethyl acetate, acetone, a mixed solvent of ethanol and diisopropyl ether, a mixed solvent of ethyl acetate and acetone, a mixed solvent of water and acetone (hereinafter, also referred to as aqueous acetone), or a mixed solvent of water and ethyl acetate (hereinafter, also referred to as aqueous ethyl acetate).

When aqueous acetone is used as the solvent in the salt resolution, its water content is not particularly limited, and is preferably 3% to 90%, more preferably 4% to 70%. When aqueous ethyl acetate is used as the solvent, its water content is not particularly limited, and is preferably 0.1% to 3%, more preferably 0.5% to 3%.

The amount of the solvent in the salt resolution is not particularly limited, and is preferably 5 times to 30 times (v/w), more preferably 5 times to 10 times (v/w) that of compound I.

The crystallization temperature in the salt resolution differs depending on the solvent used, and is -10°C to the boiling point of the solvent, preferably 0°C to 60°C. The temperature may be kept constant or may be maintained for a few hours at a temperature to deposit crystals, followed by cooling in stages. The cooling in stages is preferably performed, for example, by maintaining at 40°C to 60°C for 2 to 6 hours, followed by slow cooling (e.g., at a rate of 5 to 10°C/hour, preferably 5°C/hour down to 20 to 40°C and 10°C/hour down to -10°C to 20°C), in terms of optical purity.

The crystallization time in the salt resolution may be in the range of 1 hour to 48 hours, and is preferably in the range of 16 hours to 30 hours.

The amount of (R)-PEA is not particularly limited, and (R)-PEA is preferably reacted in an amount of, for example, 0.5 equivalent to 2 equivalents, preferably 0.5 equivalent to 1 equivalent, with respect to compound I.

The temperature to filter the crystallized compound II is not particularly limited, and is preferably -20°C to 50°C, more preferably -10°C to 30°C.

The deposited crystals can be isolated by, for example, filtration, centrifugation, or decantation. The isolated crystals can be washed, if necessary, with an appropriate solvent.

The compound II obtained by the optical resolution of compound I using (R)-PEA can be dissolved by heating in a solvent and then recrystallized by cooling, thereby further enhancing the optical purity.

Examples of the solvent in the recrystallization include, but are not particularly limited to: water; alcohol solvents such as methanol, ethanol, and isopropyl alcohol; ether solvents such as diethyl ether, dipropyl ether, diisopropyl ether, tetrahydrofuran, methyl t-butyl ether, and cyclopentyl methyl ether; ester solvents such as methyl formate, ethyl formate, methyl acetate, ethyl acetate, propyl acetate, butyl acetate, and phenyl acetate; halogenated hydrocarbon solvents such as dichloromethane, chloroform, carbon tetrachloride, dichloroethane, and tetrachloroethane; ketone solvents such as acetone, methyl ethyl ketone, diethyl ketone, and methyl isobutyl ketone; aromatic hydrocarbon solvents such as benzene, chlorobenzene, toluene, and xylene; and nitrogen-containing solvents such as acetonitrile, N,N-dimethylformamide, N,N-dimethylacetamide, and N-methylpyrrolidone. The solvent is preferably water, methanol, ethanol, isopropyl alcohol, diisopropyl ether, ethyl acetate, chloroform, acetone, toluene, acetonitrile, or a mixed solvent thereof, more preferably ethyl acetate, acetone, a mixed solvent of ethanol and diisopropyl ether, a mixed solvent of ethyl acetate and acetone, aqueous acetone, or aqueous ethyl acetate. When aqueous acetone is used, its water content is not particularly limited, and is preferably 3% to 90%, more preferably 4% to 70%. When aqueous ethyl acetate is used as the solvent, its water content is not particularly limited, and is preferably 0.1% to 3%, more preferably 0.5% to 3%. The solvent used in the recrystallization may be a solvent of a different kind from that used in the salt resolution, and is preferably a solvent of the same kind as that.

The amount of the solvent in the recrystallization is not particularly limited, and is preferably 5 times to 30 times (v/w), more preferably 5 times to 10 times (v/w) that of compound II.

The crystallization temperature in the recrystallization differs depending on the solvent used, and is -10°C to the boiling point of the solvent, preferably 0°C to 60°C. The temperature may be kept constant or may be maintained for a few hours at a temperature to deposit crystals, followed by cooling in stages. The cooling in stages is preferably performed, for example, by maintaining at 40°C to 60°C for 2 to 6 hours, followed by slow cooling (e.g., at a rate of 5 to 10°C/hour, preferably 5°C/hour down to 20 to 40°C and 10°C/hour down to -10°C to 20°C), in terms of optical purity.

The crystallization time in the recrystallization may be in the range of 1 hour to 48 hours, and is preferably in the range of 16 hours to 30 hours.

The temperature to filter the compound II crystallized by the recrystallization is not particularly limited, and is preferably -20°C to 50°C, more preferably -10°C to 30°C.

The number of recrystallization steps is not particularly limited as long as the compound of interest is obtained at favorable purity and in favorable yield. According to the process of the present invention, a highly pure compound II can be obtained by a number of recrystallization steps as exceedingly few as at least 5 or fewer, preferably 3 or fewer, more preferably 2 or fewer. Thus, the process of the present invention is very useful as a process for industrially producing compound II, as well as compound A obtained using compound II as described below in detail, and furthermore, a compound that is useful as an activated factor X inhibitor described in, for example, Patent Literature 1 to 4.

### (Step b)

Compound A can be obtained by allowing an acid such as hydrochloric acid or sulfuric acid to act on compound II (Step b).

Examples of the acid used in Step (b) include, but are not particularly limited to, hydrochloric acid, sulfuric acid, benzenesulfonic acid, methanesulfonic acid, and p-toluenesulfonic acid. The acid is preferably hydrochloric acid or sulfuric acid.

Examples of the solvent used in Step (b) include, but are not particularly limited to: water; alcohol solvents such as methanol, ethanol, and isopropyl alcohol; ether solvents such as diethyl ether, dipropyl ether, diisopropyl ether, tetrahydrofuran, methyl t-butyl ether, and cyclopentyl methyl ether; ester solvents such as methyl formate, ethyl formate, methyl acetate, ethyl acetate, propyl acetate, butyl acetate, and phenyl acetate; halogenated hydrocarbon solvents such as dichloromethane, chloroform, carbon tetrachloride, dichloroethane, and tetrachloroethane; ketone solvents such as acetone, methyl ethyl ketone, diethyl ketone, and methyl isobutyl ketone; aromatic hydrocarbon solvents such as benzene, chlorobenzene, toluene, and xylene; and nitrogen-containing solvents such as acetonitrile, N,N-dimethylformamide, N,N-dimethylacetamide, and N-methylpyrrolidone. The solvent is preferably diisopropyl ether, methyl t-butyl ether, cyclopentyl methyl ether, ethyl acetate, chloroform, dichloromethane, toluene, or a mixed solvent thereof, more preferably ethyl acetate, dichloromethane, or toluene.

The amount of the solvent used in Step (b) is not particularly limited, and is preferably 5 times to 30 times (v/w), more preferably 5 times to 10 times (v/w) that of compound II.

The reaction temperature used in Step (b) differs depending on the solvent used, and is -78°C to the boiling point of the solvent, preferably 0°C to 30°C.

The reaction time used in Step (b) may be in the range of 10 minutes to 24 hours, and is preferably in the range of 15 minutes to 8 hours.

The compound A thus synthesized is useful as an intermediate for a compound that is useful as an activated factor X (FXa) inhibitor described in, for example, Patent Literature 1 to 4.

### (Steps c, d, and e)

Compound I can be obtained by: reacting a compound represented by the general formula (III) (hereinafter, also referred to as compound III) with a C1 to C6 alkyl alcohol in the presence of an acid catalyst to obtain a compound represented by the general formula (IV) (hereinafter, also referred to as compound IV) (Step c); reacting compound IV with a base in a solvent to obtain an ester represented by the formula (V) (hereinafter, referred to as compound V) (Step d); and subsequently hydrolyzing compound V in a C1 to C6 alkyl alcohol (Step e).

Examples of the acid catalyst used in Step (c) include, but are not particularly limited to, hydrochloric acid, sulfuric acid, benzenesulfonic acid, methanesulfonic acid, and p-toluenesulfonic acid. The acid catalyst is preferably hydrochloric acid or sulfuric acid.

Examples of the C1 to C6 alkyl alcohol used in Step (c) include, but are not particularly limited to, methanol, ethanol, propanol, and isopropyl alcohol. The C1 to C6 alkyl alcohol is preferably methanol or ethanol.

The solvent used in Step (c) is not particularly limited as long as it dissolves the starting materials to some extent and does not inhibit the reaction. Examples thereof include: water; alcohol solvents such as methanol, ethanol, and isopropyl alcohol; ether solvents such as diethyl ether, dipropyl ether, diisopropyl ether, tetrahydrofuran, methyl t-butyl ether, and cyclopentyl methyl ether; ester solvents such as methyl formate, ethyl formate, methyl acetate, ethyl acetate, propyl acetate, butyl acetate, and phenyl acetate; halogenated hydrocarbon solvents such as dichloromethane, chloroform, carbon tetrachloride, dichloroethane, and tetrachloroethane; ketone solvents such as acetone, methyl ethyl ketone, diethyl ketone, and methyl isobutyl ketone; aromatic hydrocarbon solvents such as benzene, chlorobenzene, toluene, and xylene; and nitrogen-containing solvents such as acetonitrile, N,N-dimethylformamide, N,N-dimethylacetamide, and N-methylpyrrolidone. The solvent is preferably methanol or ethanol.

The amount of the solvent used in Step (c) is not particularly limited, and is preferably 5 times to 30 times (v/w), more preferably 5 times to 10 times (v/w) that of compound III.

The reaction temperature in Step (c) differs depending on the solvent used, and is -78°C to the boiling point of the solvent, preferably room temperature to the boiling point of the solvent.

The reaction time in Step (c) may be in the range of 1 hour to 24 hours, and is preferably in the range of 3 hours to 20 hours.

The solvent used in Step (d) is not particularly limited as long as it dissolves the starting materials to some extent and does not inhibit the reaction. Examples thereof include: water; alcohol solvents such as methanol, ethanol, and isopropyl alcohol; ether solvents such as diethyl ether, dipropyl ether, diisopropyl ether, tetrahydrofuran, methyl t-butyl ether, and cyclopentyl methyl ether; ester solvents such as methyl formate, ethyl formate, methyl acetate, ethyl acetate, propyl acetate, butyl acetate, and phenyl acetate; halogenated hydrocarbon solvents such as dichloromethane, chloroform, carbon tetrachloride, dichloroethane, and tetrachloroethane; ketone solvents such as acetone, methyl ethyl ketone, diethyl ketone, and methyl isobutyl ketone; aromatic hydrocarbon solvents such as benzene, chlorobenzene, toluene, and xylene; and nitrogen-containing solvents such as acetonitrile, N,N-dimethylformamide, N,N-dimethylacetamide, and N-methylpyrrolidone. The solvent is preferably N,N,-dimethylformamide, N,N,-dimethylacetamide, or N-methylpyrrolidone.

The amount of the solvent used in Step (d) is not particularly limited, and is preferably 1 time to 30 times (v/w), more preferably 5 times to 10 times (v/w) that of compound III.

Examples of the base used in Step (d) include, but are not particularly limited to: hydroxides, carbonates, bicarbonates, or alkoxides of alkali metals such as sodium, potassium, or lithium, or alkaline earth metals such as magnesium or calcium; metal hydrides such as sodium hydride, potassium hydride, and lithium hydride; alkyllithium reagents such as n-butyllithium and methyllithium; and basic heterocyclic compounds such as 1,8-diazabicyclo[5.4.0]undec-7-ene (DBU), 1,5-diazabicyclo[4.3.0]non-5-ene (DBN), and dimethylaniline. Moreover, this step may be performed in the presence of, for example, a quaternary ammonium salt (e.g., tetrabutylammonium bromide or benzyltriethylammonium chloride) or an alkali metal or alkaline earth metal iodide (e.g., potassium iodide or sodium iodide) or a crown ether, for promoting the reaction.

Of these bases, alkoxides, metal hydrides, or basic heterocyclic compounds are preferable, and sodium ethoxide, sodium hydride, or DBU is more preferable.

The amount of the base used in Step (d) is not particularly limited, and is preferably 1 equivalent to 30 equivalents, more preferably 1 equivalent to 5 equivalents, with respect to compound III.

The reaction temperature in Step (d) differs depending on the solvent used, and is -78°C to the boiling point of the solvent, preferably 50°C to the boiling point of the solvent.

The reaction time in Step (d) may be in the range of 1 hour to 24 hours, and is preferably in the range of 6 hours to 20 hours.

The hydrolysis in Step (e) is performed using an acid or an alkali. An acid such as hydrochloric acid or sulfuric acid is used in the acidic hydrolysis. A base such as an alkali metal hydroxide (e.g., sodium hydroxide or potassium hydroxide), an alkali metal carbonate (e.g., sodium carbonate or potassium carbonate), or an alkali metal bicarbonate (e.g., sodium bicarbonate or potassium bicarbonate) is used in the alkaline hydrolysis. The base is usually used in the form of an aqueous solution. Of these hydrolysis methods, alkaline hydrolysis is preferable.

The solvent used in Step (e) is not particularly limited as long as it dissolves the starting materials to some extent and does not inhibit the reaction. Examples thereof include: water; alcohol solvents such as methanol, ethanol, and isopropyl alcohol; ether solvents such as diethyl ether, dipropyl ether, diisopropyl ether, tetrahydrofuran, methyl t-butyl ether, and cyclopentyl methyl ether; ester solvents such as methyl formate, ethyl formate, methyl acetate, ethyl acetate, propyl acetate, butyl acetate, and phenyl acetate; halogenated hydrocarbon solvents such as dichloromethane, chloroform, carbon tetrachloride, dichloroethane, and tetrachloroethane; ketone solvents such as acetone, methyl ethyl ketone, diethyl ketone, and methyl isobutyl ketone; aromatic hydrocarbon solvents such as benzene, chlorobenzene, toluene, and xylene; and nitrogen-containing solvents such as acetonitrile, N,N-dimethylformamide, N,N-dimethylacetamide, and N-methylpyrrolidone. The solvent is preferably water, methanol, ethanol, isopropyl alcohol, acetonitrile, N,N,-dimethylformamide, N,N,-dimethylacetamide, or N-methylpyrrolidone, more preferably methanol, ethanol, or isopropyl alcohol.

The amount of the base used in Step (e) is not particularly limited, and is preferably 1 equivalent to 30 equivalents, more preferably 1 equivalent to 5 equivalents, with respect to compound III.

The reaction temperature in Step (e) differs depending on the solvent used, and is -78°C to the boiling point of the solvent, preferably 50°C to the boiling point of the solvent.

The reaction time in Step (e) may be in the range of 1 hour to 24 hours, and is preferably in the range of 6 hours to 20 hours.

### (Step f)

Compound I can also be obtained by reacting compound III with a base in a solvent.

The solvent used in this step is not particularly limited as long as it dissolves the starting materials to some extent and does not inhibit the reaction. Examples thereof include: water; alcohol solvents such as methanol, ethanol, and isopropyl alcohol; ether solvents such as diethyl ether, dipropyl ether, diisopropyl ether, tetrahydrofuran, methyl t-butyl ether, and cyclopentyl methyl ether; ester solvents such as methyl formate, ethyl formate, methyl acetate, ethyl acetate, propyl acetate, butyl acetate, and phenyl acetate; halogenated hydrocarbon solvents such as dichloromethane, chloroform, carbon tetrachloride, dichloroethane, and tetrachloroethane; ketone solvents such as acetone, methyl ethyl ketone, diethyl ketone, and methyl isobutyl ketone; aromatic hydrocarbon solvents such as benzene, chlorobenzene, toluene, and xylene; and nitrogen-containing solvents such as acetonitrile, N,N-dimethylformamide, N,N-dimethylacetamide, and N-methylpyrrolidone. The solvent is preferably a nitrogen-containing solvent, more preferably N,N,-dimethylformamide.

The amount of the solvent used in this step is not particularly limited, and is preferably 1 time to 50 times (v/w), more preferably 5 times to 10 times (v/w) that of compound III.

Examples of the base used in this step include, but are not particularly limited to: hydroxides, carbonates, bicarbonates, or alkoxides of alkali metals such as sodium, potassium, or lithium, or alkaline earth metals such as magnesium or calcium; metal hydrides such as sodium hydride, potassium hydride, and lithium hydride; alkyllithium reagents such as n-butyllithium and methyllithium; and basic heterocyclic compounds such as 1,8-diazabicyclo[5.4.0]undec-7-ene (DBU), 1,5-diazabicyclo[4.3.0]non-5-ene (DBN), and dimethylaniline. Of these bases, metal hydrides are preferable, and sodium hydride is more preferable.

The amount of the base used in this step is not particularly limited, and is preferably 0.1 equivalent to 10 equivalents, more preferably 1 equivalent to 5 equivalents, with respect to compound III.

The reaction temperature in this step differs depending on the solvent used, and is -78°C to the boiling point of the solvent, preferably 50°C to the boiling point of the solvent.

The reaction time in this step may be in the range of 1 hour to 24 hours, and is preferably in the range of 6 hours to 20 hours.

Thus, according to the process of the present invention, an unnecessary stereoisomer compound III obtained in Step (a) can be converted to compound I and used again in the step of obtaining compound II and compound A, as well as a compound that is useful as an activated factor X inhibitor described in, for example, Patent Literature 1 to 4. Thus, the process of the present invention is an environmentally friendly excellent process with little waste.

Hereinafter, Examples will be described. However, the present invention is not intended to be limited to them.

The optical purity (% ee) of the compounds obtained was determined as follows:

The optical purity (% ee) of the carboxylic acid in a diastereomeric salt was determined after conversion to the corresponding carboxylic acid. The optical purity (% ee) of the carboxylic acid, the methyl ester, and the ethyl ester was determined by GC.
Optical purity analysis conditions; detector: FID, column: J&W Cyclodex (registered trademark), 30 mx0.25 mm, sample vaporizing chamber temperature: 250°C, column temperature: 90°C, detecting unit temperature: 250°C, carrier gas: helium, flow rate: 1 ml/min.

### Examples

### (Example 1) (R)-α-Phenylethylamine salt of (S)-3-cyclohexene-1-carboxylic acid

3-Cyclohexene-1-carboxylic acid (1.0 kg) was dissolved in 4.8% aqueous acetone (7.5 L). To the solution, a solution of (R)-α-phenylethylamine (624.3 g) dissolved in 4.8% aqueous acetone (500 ml) was gradually added at 50°C, and the mixture was stirred at this temperature for 4 hours. The suspension was cooled to 35°C and stirred at this temperature for 16 hours and then further at 10°C for 3 hours. The suspension was subjected to filtration under reduced pressure to obtain 837.1 g of the title compound as white crystals. Its optical purity was 63% de. To the obtained salt (700 g), 4.8% aqueous acetone (5.6 L) was subsequently added, and the mixture was stirred for 5 hours under heating to reflux, at 30°C for 13 hours, and then for 3 hours under ice cooling. The suspension was subjected to filtration under reduced pressure to obtain 519.4 g of the title compound as white crystals. Its optical purity was 81% de. To the obtained salt (500 g), 4.8% aqueous acetone (4.0 L) was further added, and the mixture was stirred for 5 hours under heating to reflux, at 30°C for 13 hours, and then at 10°C for 3 hours. The suspension was subjected to filtration under reduced pressure to obtain 398.5 g of the title compound as white crystals. Its optical purity was 91% de. Finally, to the obtained salt (300 g), 4.8% aqueous acetone (2.4 L) was added, and the mixture was stirred for 5 hours under heating to reflux, at 30°C for 13 hours, and then at 10°C for 3 hours. The suspension was subjected to filtration under reduced pressure to obtain 240.0 g of the title compound as white crystals. Its optical purity was 97% de.
1H-NMR (D2O) δ: 1.50-1.63 (1H, m), 1.66 (3H, d, J = 6.9 hz), 1.86-1.95 (1H, m), 1.98-2.25 (4H, m), 2.32-2.43 (1H, m), 4.56 (1H, q, J = 6.9Hz), 5.70-5.80 (2H, m), 7.40-7.55 (5H, m)
Anal.: C15H21N1O2
Theoretical (%) C; 72.84, H; 8.56, N; 5.66
Found (%) C; 72.88, H; 8.58, N; 5.72

### (Example 2) (R)-α-Phenylethylamime salt of (S)-3-cyclohexene-1-carboxylic acid

3-Cyclohexene-1-carboxylic acid (30 g) was dissolved in 3% aqueous ethyl acetate (150 ml). To the solution, a solution of (R)-α-phenylethylamine (23.0 g) dissolved in 3% aqueous ethyl acetate (30 ml) was gradually added at 55°C, and the mixture was stirred at this temperature for 6 hours. The suspension was stirred at 25°C for 5 hours and further at -10°C for 2.5 hours. The suspension was subjected to filtration under reduced pressure to obtain 32.9 g of the title compound as white crystals. Its optical purity was 49% de. To the obtained salt (32.7 g), 3% aqueous ethyl acetate (196 ml) was subsequently added, and the mixture was stirred at 55°C for 3 hours, then at 25°C for 5 hours, and further at -10°C for 2.5 hours. The suspension was subjected to filtration under reduced pressure to obtain 24.7 g of the title compound as white crystals. Its optical purity was 78% de. To the obtained salt (24.6 g), 3% aqueous ethyl acetate (148 ml) was further added, and the mixture was stirred at 55°C for 3 hours, then at 25°C for 5 hours, and further at -10°C for 2.5 hours. The suspension was subjected to filtration under reduced pressure to obtain 20.3 g of the title compound as white crystals. Its optical purity was 95% de.
Various spectroscopic data were in agreement with those of Example 1.

### (Reference Example 1) (R)-α-Phenylethylamine salt of (S)-3-cyclohexene-1-carboxylic acid

3-Cyclohexene-1-carboxylic acid (10.0 g) was dissolved in acetone (70 ml). To the solution, a solution of (R)-α-phenylethylamine (6.2 g) in acetone (10 ml) was gradually added at 50°C, and the mixture was stirred at this temperature for hours. The suspension was cooled to 30°C and stirred at this temperature for 16 hours and then further at 10°C for 3 hours. The suspension was subjected to filtration under reduced pressure to obtain 9.5 g of the title compound as white crystals. Its optical purity was 45% de. Various spectroscopic data were in agreement with those of Example 1.

### (Reference Example 2) (R)-α-Phenylethylamine salt of (S)-3-cyclohexene-1-carboxylic acid

3-Cyclohexene-1-carboxylic acid (10.0 g) was dissolved in ethyl acetate (50 ml). To the solution, a solution of (R)-α-phenylethylamine (6.2 g) in ethyl acetate (10 ml) was gradually added at 50°C, and the mixture was stirred at this temperature for 4 hours. The suspension was cooled to 30°C and stirred at this temperature for 16 hours and then further at 10°C for 3 hours. The suspension was subjected to filtration under reduced pressure to obtain 9.8 g of the title compound as white crystals. Its optical purity was 40% de.
Various spectroscopic data were in agreement with those of Example 1.

### (Example 3) (S)-3-Cyclohexene-1-carboxylic acid

To the (R)-α-phenylethylamine salt of (S)-3-cyclohexene-1-carboxylic acid (1.0 g, 97% de), methyl t-butyl ether (20 ml) and 1 N hydrochloric acid solution were added until the pH of the solution became 1. The mixture was stirred at room temperature for 1 hour. The organic layer was dried over anhydrous magnesium sulfate, and the solvent was then distilled off to obtain 504 mg of the title compound as a colorless oil.
1H-NMR (CDCl3) δ: 1.64-1.75 (1H, m), 1.99-2.20 (3H, m), 2.24-2.30 (2H, m), 2.56-2.63 (1H, m), 5.63-5.70 (2H, m)

### (Example 4) Methyl (R)-3-cyclohexene-1-carboxylate

(R)-3-Cyclohexene-1-carboxylic acid (1.0 g, 97% de) was dissolved in methanol (10 ml), and 5 N aqueous hydrochloric acid solution (1 ml) was added to the solution at room temperature. The reaction solution was heated to reflux for 6 hours, and the solvent was then distilled off. To the obtained residue, methyl t-butyl ether was added, and the organic layer was then washed with saturated sodium bicarbonate solution and water, and then dried over anhydrous magnesium sulfate. The solvent was distilled off, and the obtained residue was then subjected to silica gel column chromatography (ethyl acetate-normal hexane=1:1) to obtain 1.08 g of the title compound as a colorless oil. Its optical purity was 97% ee.
1H-NMR (CDCl3) δ: 1.60-1.77 (1H, m), 1.95-2.13 (3H, m), 2.23-2.29 (2H, m), 2.50-2.62 (1H, m), 3.70 (3H, s), 5.64-5.71 (2H, m)

### (Example 5) Ethyl (R)-3-cyclohexene-1-carboxylate

(R)-3-Cyclohexene-1-carboxylic acid (1.0 g, 97% de) was dissolved in ethanol (10 ml), and 5 N aqueous hydrochloric acid solution (1 ml) was added to the solution at room temperature. The reaction solution was heated to reflux for 6 hours, and the solvent was then distilled off. To the obtained residue, methyl t-butyl ether was added, and the organic layer was then washed with saturated sodium bicarbonate solution and water, and then dried over anhydrous magnesium sulfate. The solvent was distilled off, and the obtained residue was then subjected to silica gel column chromatography (ethyl acetate-normal hexane=1:1) to obtain 1.13 g of the title compound as a colorless oil. Its optical purity was 97% ee.
1H-NMR (CDCl3) δ: 1.26 (3H, t, J = 7.2Hz), 1.62-1.75 (1H, m), 1.95-2.15 (3H, m), 2.21-2.30 (2H, m), 2.49-2.59 (1H, m), 4.14 (2H, q, J = 7.2 Hz), 5.64-5.72 (2H, m)

### (Example 6) Methyl-3-cyclohexene-1-carboxylate

Methyl (R)-3-cyclohexene-1-carboxylate (1.0 g, 97% ee) was dissolved in N,N-dimethylformamide (10 ml). To the solution, 1,8-diazabicyclo[5.4.0]undec-7-ene (1.1 ml) was added at room temperature, and the mixture was stirred at 120°C for 18 hours. The reaction solution was cooled to room temperature, and 10% aqueous citric acid solution was then added dropwise thereto, followed by extraction with cyclopentyl methyl ether. The organic layer was washed with water and then dried over anhydrous magnesium sulfate. The solvent was distilled off, and the obtained residue was then subjected to silica gel column chromatography (ethyl acetate-normal hexane=1:1) to obtain 0.91 g of the title compound as a colorless oil. Its optical purity was 0% ee. The H-NMR spectroscopic data were in agreement with those of Example 4.

### (Example 7) Ethyl-3-cyclohexene-1-carboxylate

Ethyl (R)-cyclohexene-1-carboxylate (1.0 g, 97% ee) was dissolved in N,N-dimethylformamide (10 ml). To the solution, 1,8-diazabicyclo[5.4.0]undec-7-ene (1.0 ml) was added at room temperature, and the mixture was stirred at 120°C for 18 hours. The reaction solution was cooled to room temperature, and 10% aqueous citric acid solution was then added dropwise thereto, followed by extraction with cyclopentyl methyl ether. The organic layer was washed with water and then dried over anhydrous magnesium sulfate. The solvent was distilled off, and the obtained residue was then subjected to silica gel column chromatography (ethyl acetate-normal hexane=1:1) to obtain 0.89 g of the title compound as a colorless oil.
Its optical purity was 0% ee. The H-NMR spectroscopic data were in agreement with those of Example 5.

### (Example 8) 3-Cyclohexene-1-carboxylic acid

Methyl-3-cyclohexene-1-carboxylate (1.0 g) was dissolved in methanol (10 ml). To the solution, 5 N aqueous sodium hydroxide solution (5 ml) was added at room temperature, and the mixture was stirred at this temperature for 16 hours. Hydrochloric acid was added to the reaction solution, followed by extraction with cyclopentyl methyl ether. The organic layer was washed with water and then dried over anhydrous magnesium sulfate. The solvent was distilled off, and the obtained residue was then subjected to silica gel column chromatography (chloroform-methanol=3:1) to obtain 855 mg of the title compound as a colorless oil. The H-NMR spectroscopic data were in agreement with those of Example 3.

### (Example 9) 3-Cyclohexene-1-carbaxylic acid

Ethyl-3-cyclohexene-1-carboxylate (1.0 g) was dissolved in ethanol (10 ml). To the solution, 5 N aqueous sodium hydroxide solution (5 ml) was added at room temperature, and the mixture was stirred at this temperature for 16 hours. Hydrochloric acid was added to the reaction solution, followed by extraction with cyclopentyl methyl ether. The organic layer was washed with water and then dried over anhydrous magnesium sulfate. The solvent was distilled off, and the obtained residue was then subjected to silica gel column chromatography (chloroform-methanol=3:1) to obtain 800 mg of the title compound as a colorless oil. The H-NMR spectroscopic data were in agreement with those of Example 3.

### (Example 10) 3-Cyclohexene-1-carboxylic acid

(R)-3-Cyclohexene-1-carboxylic acid (1.0 g, 97% ee) was dissolved in N,N-dimethylformamide (10 ml). To the solution, 60% sodium hydride (634 mg) was added at room temperature, and the mixture was stirred at 120°C for 18 hours. The reaction solution was cooled to room temperature, and 10% aqueous citric acid solution was then added dropwise thereto, followed by extraction with cyclopentyl methyl ether. The organic layer was washed with water and then dried over anhydrous magnesium sulfate. The solvent was distilled off, and the obtained residue was then subjected to silica gel column chromatography (chloroform-methanol=3:1) to obtain 892 mg of the title compound as a colorless oil. Its optical purity was 0% ee. The H-NMR spectroscopic data were in agreement with those of Example 3.

## Claims

1. A process for producing a compound represented by the general formula (II): 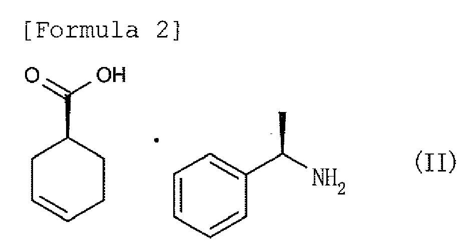 the process comprising reacting a compound represented by the general formula (I): 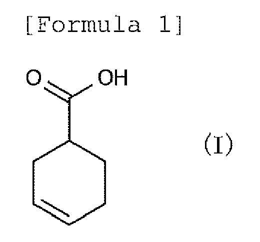 with (R)-α-phenylethylamine using aqueous acetone or aqueous ethyl acetate as a solvent.

2. The process according to claim 1, further comprising the step of recrystallizing the compound represented by the general formula (II) using aqueous acetone or aqueous ethyl acetate as a recrystallization solvent.

3. The process according to claim 2, wherein aqueous ethyl acetate is used as the solvent and the recrystallization solvent.

4. The process according to claim 3, wherein the aqueous ethyl acetate has a water content of 0.5% to 3.0%.

5. The process according to any one of claims 1 to 4, further comprising the step of treating the compound represented by the general formula (II) with an acid to obtain a compound represented by the general formula (A):

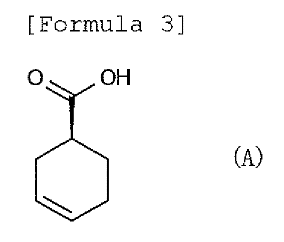

6. A process for producing a compound represented by the general formula (I): 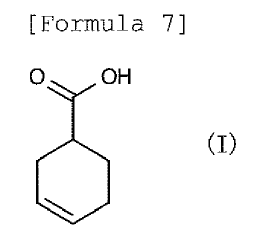 the process comprising: reacting a stereoisomer represented by the general formula (II): 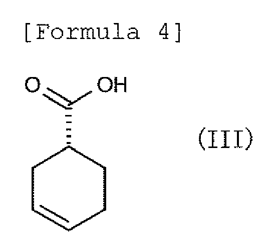 obtained in a production process according to claim 1, with a C1 to C6 alkyl alcohol in the presence of an acid catalyst;
reacting the obtained compound represented by the general formula (IV): 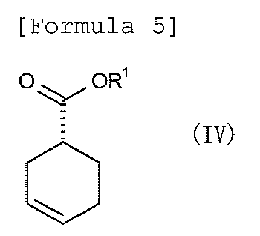 (wherein R¹ represents a C1 to C6 alkyl group) with a base in a solvent to obtain an ester represented by the general formula (V): 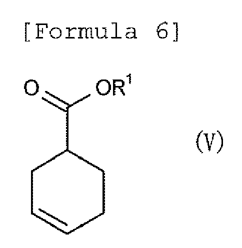 (wherein R¹ is as defined above); and
hydrolyzing the ester in a C1 to C6 alkyl alcohol.

7. The process according to claim 6, wherein the solvent used in the step of obtaining the compound represented by the general formula (V) from the compound represented by the general formula (IV) is N,N-dimethylformamide.

8. The process according to claim 6 or 7, wherein the base used in the step of obtaining the compound represented by the general formula (V) from the compound represented by the general formula (IV) is 1,8-diazabicyclo[5.4.0undec-7-ene.

9. The process according to any one of claims 6 to 8, wherein the solvent used in the hydrolysis is methanol or ethanol.

10. The process according to any one of claims 6 to 9, wherein a base used in the hydrolysis is sodium hydroxide.

11. A process for producing a compound represented by the general formula (I): 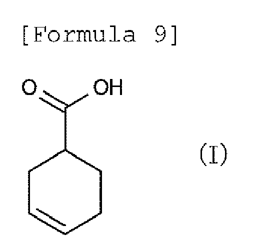 the process comprising reacting a stereoisomer represented by the general formula (III): 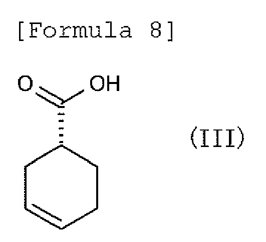 obtained in a production process according to claim 1, with a base in a solvent.

12. The process according to claim 11, wherein the solvent is N,N-dimethylformamide.

13. The process according to claim 11 or 12, wherein the base is sodium hydride.
